# EUROPEAN PATENT APPLICATION

(11) **EP 1 293 779 A2**
(43) Date of publication of application: **19.03.2003**
(21) Application number: 02019719.0
(22) Date of filing: 03.09.2002
(51) Int. Cl.: G01N 33/543, B05D 1/18

(54) **Biosensor surface**

(30) Priority: 03.09.2001 JP 2001265484
(71) Applicant: FUJI PHOTO FILM CO., LTD., Kanagawa-ken, 250-0193 (JP)
(72) Inventor: Yang, Bo, c/o Hitec Co., Ltd., Yokohama R&D Center, Nishi-ku, Yokohama-shi Kanagawa 220-6012 (JP); Kageyama, Shigeki, Asaka-shi, Saitama 351-8585 (JP); Kojima, Masayoshi, Asaka-shi, Saitama 351-8585 (JP); Ito, Toshihisa, Asaka-shi, Saitama 351-8585 (JP); Shinoki, Hiroshi, Asaka-shi, Saitama 351-8585 (JP); Abe, Yoshihiko, Asaka-shi, Saitama 351-8585 (JP)
(74) Representative: Albrecht, Thomas, Dr.

(57) **Abstract**

The present invention provides a biosensor surface comprising a metal surface or metal membrane treated with a linker compound capable of binding to a physiologically active substance, a measurement chip for a biosensor having a physiologically active substance immobilized on the biosensor surface, a method for producing the same, and a method for measurement using the same. The present invention can provide a method for immobilizing a physiologically active substance on a metal surface in simple processes with high reliability while most of the molecules maintain activity and do not become detached.

## Description

### Technical Field of the Invention

The present invention relates to a biosensor surface comprising a metal surface or metal membrane treated with a linker compound capable of binding to a physiologically active substance, a measurement chip for a biosensor having a physiologically active substance immobilized on the biosensor surface, a method for producing the same, and a method for measurement using the same.

### Background Art

Recently, a large number of measurements using intermolecular interactions such as immune response are carried out in the clinical tests etc. Since conventional methods require a complicated operation or a labeling substance, several techniques have been used that are capable of detecting changes in the binding level of a substance to be measured with high sensitivity and without the need for a labeling substance. Examples thereof are: a surface plasmon resonance (SPR) measuring technique; a quartz crystal microbalance (QCM) measuring technique; and a measuring technique employing functionalized surfaces of particles ranging from gold colloid particles to ultrafine particles. The SPR measuring technique is a method for detecting adsorption and desorption occurring in the vicinity of the surface based on refractive index variation or peak shift in the vicinity of an organic functional membrane which is in contact with a metal membrane of a chip. The QCM measuring technique is capable of detecting a mass of adsorption and desorption at a level of "ng" based on change in oscillation frequency of an oscillator caused by adsorption and desorption of a substance on a gold electrode (device) of a crystal oscillator. Also, a surface of gold ultrafine particles (nm level) is functionalized and physiologically active substances are immobilized thereon, and a specific recognition reaction between physiologically active substances can be carried out. Thus, an organism-related substance can be detected based on sedimentation and arrangement of gold fine particles.

A surface on which physiologically active substances are immobilized is important in all the above techniques. Surface plasmon resonance (SPR), which is most widely used in the art, is hereinafter described as an example.

A commonly used measurement chip comprises a transparent substrate (such as glass), a vapor-deposited metal membrane, and a thin film having a functional group capable of immobilizing a physiologically active substance thereon. A physiologically active substance is immobilized on the metal surface via the functional group. By measuring a specific binding reaction between the physiologically active substance and an analyte, the interaction between biomolecules is analyzed.

As a thin film having a functional group capable of immobilizing a physiological active substance, there has been reported a measurement chip which has a physiologically active substance immobilized thereon by using a compound having a functional group binding to a metal, a linker having a chain length of 10 or more atoms, and a functional group capable of binding to a physiologically active substance (see Japanese Patent No. 2815120).

When a physiologically active substance is immobilized on a metal membrane, however, the physiologically active substance is immobilized not only via relevant covalent binding but also by partial physical adsorption. When a protein is immobilized on a gold surface by physical adsorption, it is likely to be denatured. In this case, unlike with immobilization via covalent binding, there is a problem that proteins become detached during measurement. These problems eventually result in lowered sensitivity and deteriorated reproducibility of the measurement. Therefore, means for eliminating unnecessary physical adsorption have been sought.

For this purpose, a method has been used in which a hydrophilic hydrogel is immobilized on a metal surface via a linker to inhibit physical adsorption (see Japanese Patent No. 2815120, U.S. Patent No. 5436161, and Japanese Patent Application Laying-Open No. 8-193948). In this method, however, the hydrogel was not easily immobilized, and thus, this method was not suitable for various users to perform surface treatment for their intended purposes. Further, where a hydrogel is used, when a high molecular weight analyte such as a cell is measured, there is a problem that the analyte can not enter into a gap of the matrix.

In contrast, as a method for reducing non-specific binding of physiologically active substances by physical adsorption, so-called blocking by bovine serum albumin and the like was carried out in the immunochemical analysis using a plastic plate. In the analysis using a metal surface, it is reported that, when a DNA sensor using SPR is prepared by immobilizing DNA, the terminus of which was subjected to thiolization, on the metal membrane, the DNA of interest can be detected with high sensitivity without causing non-specific adsorption by adding 6-hydroxy-1-hexanethiol (see U.S. Patent No. 5942397 and J. Am. Chem. Soc., 1997, 119, 8916-8920). It is also reported that a thiolized biotin and 11-hydroxy-1-undecanethiol are simultaneously mixed, and the reaction with streptoavidin is detected with non-specific binding being inhibited (see J. Am. Chem. Soc., 1999, 121, 6469-6478). However, these are directed to inhibiting non-specific adsorption of the analysis subject (analyte) and thus do not solve problems associated with unnecessary denaturation or detachment of physiologically active substances caused by physical adsorption of a part of the physiologically active substance to be immobilized. Synthesis of a derivative of a physiologically active substance which can be directly immobilized on a metal surface is not easy, and it is desired to develop a metal surface on which a physiologically active substance can be immobilized without modification. These problems exist on the surface plasmon resonance (SPR) technique as well as of the QCM technique and the gold ultrafine particle technique.

### Summary of the Invention

The object of the present invention is to solve the problems of the prior art. More specifically, the object of the present invention is to provide a method for immobilizing a physiologically active substance on a metal surface in simple processes with high reliability while most of the molecules maintain activity and do not become detached.

The present inventors have conducted concentrated studies to solve the above object. As a result, they have found that a physiologically active substance can be stably immobilized on a metal surface via covalent binding by treating the metal surface with two compounds, i.e., one for binding a physiologically active substance via covalent binding and the other for inhibiting physical adsorption of the physiologically active substance, and then reacting the surface with the physiologically active substance, and they have completed the present invention. A biosensor chip prepared using this surface was also found to alleviate the problems of the prior art.

Thus, according to the present invention, there is provided a biosensor surface for immobilizing a physiologically active substance via covalent binding, which comprises a metal surface or metal membrane treated with a mixture containing at least one compound represented by formula (1) and at least one compound represented by formula (2);

X¹-A¹-Y¹ (1)

in formula (1), X¹ represents a functional group capable of covalently binding to a metal surface; A¹ represents a divalent linking group selected from a substituted or unsubstituted amino acid, an aliphatic group, an aromatic group, a heterocyclic group or a combination thereof; and Y¹ represents a functional group capable of binding to a physiologically active substance.

X²-A²-Y² (2)

in formula (2), X² represents a functional group capable of covalently binding to a metal surface; A² represents a divalent linking group selected from a substituted or unsubstituted amino acid, an aliphatic group, an aromatic group, a heterocyclic group or a combination thereof; and Y² represents a functional group capable of improving a performance of a sensor.

The biosensor surface of the present invention is preferably used in non-electrochemical detection, and is particularly preferably used in a surface plasmon resonance analysis.

Preferably, in formula (1) or (2), X¹ and X² are thiol (-SH) or asymmetric or symmetric disulfide (-SS-).

Preferably, in formula (1), Y¹ is -OH, -COOH, -NH₂, -CHO, -NHNH₂, -NCS, an epoxy group, or a vinyl group.

Preferably, in formula (2), Y² is -OH, -COOH, -NH₂, -SO₃H, a sugar, a nucleic acid, a protein, or a water-soluble polymer.

Preferably, the compound represented by formula (1) is HS-(CH₂)ₙ-COOH wherein n represents an integer of 1 to 20 or HS-(CH₂)ₙ-NH₂ wherein n represents an integer of 1 to 20.

Preferably, the compound represented by formula (2) is HS-(CH₂)ₙ-OH wherein n represents an integer of 1 to 20, HS-(CH₂)ₙ-NH₂ wherein n represents an integer of 1 to 20, HS-(CH₂)ₙ-SO₃H wherein n represents an integer of 1 to 20, a sugar or sugar derivative having a thiol group, a protein or protein derivative having a thiol group, or a nucleic acid or nucleic acid derivative having a thiol group.

According to another aspect of the present invention, there is provided a measurement chip for a biosensor which is obtained by covalently binding a physiologically active substance on the aforementioned biosensor surface of the present invention.

According to further another aspect of the present invention, there is provided a method for detecting and/or measuring a substance which interacts with a physiologically active substance immobilized on the biosensor surface, which comprises a step of bringing the aforementioned biosensor surface of the present invention or the aforementioned measurement chip for a biosensor of the present invention into contact with a test substance.

Preferably, the interaction between a physiologically active substance immobilized on the biosensor surface and the test substance is detected and/or measured by a non-electrochemical method. Particularly preferably, the interaction between a physiologically active substance immobilized on the biosensor surface and the test substance is detected and/or measured by surface plasmon resonance analysis.

According to further another aspect of the present invention, there is provided a method for producing a biosensor surface for immobilizing a physiologically active substance via covalent binding which comprises a metal surface or metal membrane treated with a mixture containing at least one compound represented by formula (1) and at least one compound represented by formula (2), which comprises a step of treating a metal surface or metal membrane with a mixture containing at least one compound represented by formula (1) and at least one compound represented by formula (2);

X¹-A¹-Y¹ (1)

in formula (1), X¹ represents a functional group capable of covalently binding to a metal surface; A¹ represents a divalent linking group selected from a substituted or unsubstituted amino acid, an aliphatic group, an aromatic group, a heterocyclic group or a combination thereof; and Y¹ represents a functional group capable of binding to a physiologically active substance.

X²-A²-Y² (2)

in formula (2), X² represents a functional group capable of covalently binding to a metal surface; A² represents a divalent linking group selected from a substituted or unsubstituted amino acid, an aliphatic group, an aromatic group, a heterocyclic group or a combination thereof; and Y² represents a functional group capable of improving a performance of a sensor.

According to further another aspect of the present invention, there is provided a method for immobilizing a physiologically active substance on a metal surface or metal membrane, which comprises steps of: treating a metal surface or metal membrane with a mixture containing at least one compound represented by formula (1) and at least one compound represented by formula (2); and covalently binding a physiologically active substance to the compound represented by formula (1) directly or via a crosslinking compound or hydrogel.

X¹-A¹-Y¹ (1)

in formula (1), X¹ represents a functional group capable of covalently binding to a metal surface; A¹ represents a divalent linking group selected from a substituted or unsubstituted amino acid, an aliphatic group, an aromatic group, a heterocyclic group or a combination thereof; and Y¹ represents a functional group capable of binding to a physiologically active substance.

X²-A²-Y² (2)

in formula (2), X² represents a functional group capable of covalently binding to a metal surface; A² represents a divalent linking group selected from a substituted or unsubstituted amino acid, an aliphatic group, an aromatic group, a heterocyclic group or a combination thereof; and Y² represents a functional group capable of improving a performance of a sensor.

### Detailed Description of the Invention

The embodiments for carrying out the present invention are described below.

The biosensor surface according to the present invention comprises a metal surface or metal membrane treated with a mixture containing at least one compound represented by formula (1) and at least one compound represented by formula (2).

X¹-A¹-Y¹ (1)

in formula (1), X¹ represents a functional group capable of covalently binding to a metal surface; A¹ represents a divalent linking group selected from a substituted or unsubstituted amino acid, an aliphatic group, an aromatic group, a heterocyclic group or a combination thereof; and Y¹ represents a functional group capable of binding to a physiologically active substance.

X²-A²-Y² (2)

in formula (2), X² represents a functional group capable of covalently binding to a metal surface; A² represents a divalent linking group selected from a substituted or unsubstituted amino acid, an aliphatic group, an aromatic group, a heterocyclic group or a combination thereof; and Y² represents a functional group capable of improving a performance of a sensor.

The biosensor surface according to the present invention is produced by treating a metal surface or metal membrane with a mixture containing at least one compound represented by formula (1) and at least one compound represented by formula (2) which are defined in the present specification.

A metal membrane is preferably located on a substrate. The term "located on a substrate" refers to the case where a metal membrane is located in such a way that it is in direct contact with the substrate, as well as the case where a metal membrane is located on the substrate via another layer without being in direct contact with the substrate.

When a metal membrane is located on a substrate, the measurement chip for a biosensor of the present invention has a substrate, a metal membrane formed on the substrate, and a linker layer formed on the metal membrane (comprising a compound represented by formula I).

Any substrate for a surface plasmon resonance biosensor can be used in the present invention, so far as it is applicable to an immobilization method. Generally, substrates that can be used herein are those made of materials transparent to a laser beam, such as glass, polyethylene terephthalate and polycarbonate. Such a substrate is preferably made of a material which is not anisotropic to polarized light, and has excellent workability. The thickness of the substrate is not particularly limited, and is normally about 0.1 to 20mm.

Examples of a metal membrane for the measurement chip for a biosensor of the present invention are not specifically limited when it is used for a surface plasmon resonance biosensor, so far as they can bring about surface plasmon resonance. Examples of a metal type that can be applied for the metal membrane include gold, silver, copper, aluminum, and platinum. These metals can be used alone or in combination. Furthermore, taking the adherence of the metal to the above substrate into account, an interstitial layer of chromium or the like may be provided between the substrate and the layer of gold, silver etc.

The thickness of the metal membrane is not particularly limited. For example, when the metal membrane is used for a surface plasmon resonance biosensor, the thickness is preferably 100 to 2,000 angstrom, and particularly preferably, 200 to 600 angstrom. When the thickness is more than 3,000 angstrom, it becomes impossible to sufficiently detect the surface plasmon phenomenon of the medium. When an interstitial layer of chromium or the like is provided, the thickness of the interstitial layer is preferably 5 to 50 angstrom.

The formation of a metal membrane may be performed according to standard techniques such as sputtering, evaporation, ion plating, electroplating and electroless plating.

The compounds represented by formula (1) and (2) used in the present invention are described in the following.

In formulas (1) and (2), types of X¹ and X² are not particularly limited as long as they are functional groups capable of covalently binding to a metal surface. X¹ and X² are preferably thiol (-SH) or asymmetric or symmetric disulfide (-SS-), and are particularly preferably thiol (-SH).

In formulas (1) and (2), A¹ and A² represent a divalent linking group selected from a substituted or unsubstituted amino acid, an aliphatic group, an aromatic group, a heterocyclic group or a combination thereof. A¹ and A² are preferably a hydrocarbon group. The divalent linking group represented by A¹ and A² preferably has a chain length of 10 or less atoms, and more preferably has a chain length of 8 or less atoms.

Suitable amino acids include glycine and alanine, and may be peptides formed by polymerization thereof.

The aliphatic group includes an alkylene group, an alkenylene group, and an alkynylene group. The form of chain may be a linear chain, a branched chain, a cyclic chain or a combination thereof. As an aliphatic group, an alkylene group is particularly preferred, and a linear alkylene group is most preferred. The length of the aliphatic group is not particularly limited, and the length is for example 1 to 20 carbon atoms, more preferably about 1 to 10 carbon atoms, and particularly preferably about 2 to 10 carbon atoms.

The aromatic group includes an arylene group, and more specifically, a phenylene group, a naphthylene group and the like.

The heterocycle includes a 5- or 7-membered saturated or unsaturated monocycle or condensed cycle comprising one or more of one or more types of hetero atoms selected from a nitrogen, oxygen or sulfur atom. More specifically, a heterocycle includes pyridine, quinoline, isoquinoline, pyrimidine, pyrazine, pyridadine, phthalazine, triazine, furan, thiophene, pyrrole, oxazole, benzoxazole, thiazole, benzothiazole, imidazole, benzimidazole, thiadiazole, and triazole. The term "heterocyclic group" means a divalent group derived from the heterocycles stated above.

A divalent linking group represented by A¹ and A² may also be constituted by the combination of the aliphatic group, the aromatic group, or the heterocyclic group as mentioned above.

In formula (1), Y¹ represents a functional group capable of binding to a physiologically active substance. The type of the functional group can be suitably selected depending on the type of a physiologically active substance to be immobilized. In general, Y¹ is -OH, -COOH, -NH₂, -CHO, -NHNH₂, -NCS, an epoxy group, a vinyl group or the like.

In formula (2), Y² represents a functional group capable of improving the performance of a sensor. Examples thereof include a functional group capable of inhibiting physical adsorption of a physiologically active substance on a metal surface. Such a functional group is different from the functional group capable of binding to a physiologically active substance represented by Y¹, and can be suitably selected depending on the type of physiologically active substance to be immobilized. In general, Y² is -OH, -COOH, -NH₂, -SO₃H, a sugar, a nucleic acid, a protein, or a water-soluble polymer (for example, a hydrophilic group such as polyoxyethylene).

According to a particularly preferred embodiment of the present invention, the compound represented by formula (1) is HS-(CH₂)ₙ-COOH or HS-(CH₂)ₙ-NH₂ wherein n represents an integer of 1 to 20, preferably an integer of 1 to 10, and more preferably an integer of 1 to 8.

According to a particularly preferred embodiment of the present invention, the compound represented by formula (2) is HS-(CH₂)ₙ-OH, HS-(CH₂)ₙ-NH₂, HS-(CH₂)ₙ-SO₃H, a sugar or sugar derivative having a thiol group, a protein or protein derivative having a thiol group, or a nucleic acid or nucleic acid derivative having a thiol group wherein n represents an integer of 1 to 20, preferably an integer of 1 to 10, and more preferably an integer of 1 to 8.

In the present invention, a physiologically active substance can be stably immobilized on a metal surface via covalent binding by treating a metal membrane with a solution obtained by mixing a compound having a functional group capable of covalently binding a physiologically active substance (i.e., a compound represented by formula (1)) and a compound having a functional group capable of improving the performance of a sensor (i.e., a compound represented by formula (2), preferably a compound capable of inhibiting physical adsorption of the physiologically active substance) at a certain ratio.

A mixing ratio (a mole ratio) of a compound represented by formula (1) to a compound represented by formula (2) can be suitably selected depending on the type of a physiologically active substance, experimental conditions and the like. The mole ratio of a compound represented by formula (1) to a compound represented by formula (2) is generally in the range of 1 : 100 to 100 : 1, and preferably in the range of 1 : 10 to 10 : 1. When the ratio of the compound represented by formula (1) is higher than this range, it is not preferred because the effect of the compound represented by formula (2) of inhibiting physical adsorption of a physiologically active substance is deteriorated. When the ratio of the compound represented by formula (1) is lower than this range, it is not preferred because the efficiency of immobilizing a physiologically active substance is deteriorated. The mixing ratio (the mole ratio) of the compound represented by formula (1) to the compound represented by formula (2) is desirably determined by considering the balance between the efficiency of immobilizing a physiologically active substance and the effect of inhibiting physical adsorption of a physiologically active substance.

In the present invention, a biosensor surface is prepared by treating a metal surface or metal membrane using a mixture of two types of compounds represented by formulas (1) and (2). More specifically, a biosensor surface can be prepared by immersing a metal surface or metal membrane in a solution obtained by mixing two types of compounds represented by formulae (1) and (2) in a suitable solvent (e.g., ethanol) in the aforementioned suitable range of mole ratio, and performing surface treatment for a given period of time.

Examples of the methods for treating a metal surface or metal membrane using a mixture of two types of compounds represented by formulae (1) and (2) include a method which immerses a metal membrane or the like in a mixed solution containing the compounds as mentioned above for a certain time (immersion method), as well as a method which uses a spin coater (spin-coating method), and a method which uses a gravure printer (gravure method).

The thus obtained biosensor surface has a compound represented by formula (1) on its surface. The present invention provides a method for immobilizing a physiologically active substance on a metal surface or metal membrane by covalently binding the physiologically active substance to a compound represented by formula (1) which is immobilized on the biosensor surface directly or via a crosslinking compound (e.g., water-soluble polyvalent reagent) or hydrogel.

Examples of a crosslinking compound include glutaraldehyde, periodic acid, N-succinimydyl-2-maleimide acetic acid, N-succinimydyl-4-maleimide butyric acid, N-succinimydyl-6-maleimide hexanoic acid,
N-succinimydyl-4-maleimidemethylcyclohexane-1-carboxylic acid,
N-sulfosuccinimydyl-4-maleimidemethylcyclohexane-1-carboxylic acid,
N-succinimydyl-4-maleimidemethyl benzoic acid, N-succinimydyl-3-maleimide benzoic acid, N-sulfosuccinimydyl-3-maleimide benzoic acid,
N-succinimydyl-4-maleimidephenyl-4-butyric acid,
N-sulfosuccinimydyl-4-maleimidephenyl-4-butyric acid,
NN'-oxydimethylene-dimaleimide, NN'-O-phenylene-dimaleimide,
N,N'-m-phenylene-dimaleimide, N,N'-p-phenylene-dimaleimide,
N,N'-hexamethylene-dimaleimide, N-succinimydylmaleimide carboxylic acid,
N-succinimydyl-S-acetylmercaptoacetic acid,
N-succinimydyl-3-(2-pyridyldithio)propionate, S-acetylmercapto succinic anhydride,
methyl-3-(4'-dithiopyridyl)propionimidate, methyl-4-mercaptobutylimidate,
methyl-3-mercaptopropionimidate, iminothiolene, O-carboxymethyl-hydroxylamine,
azodiphenylmaleimide, bis(sulfosuccinimydyl)suberate,
4,4'-diisothiocyano-2,2'-disulfonic acid stilbene,
4,4'-difluoro-3,3'-dinitrodiphenylsulfone, 1,5-difluoro-2,4-dinitrobenzene,
p-phenylenediisothiocyanate, dimethyladipimidate, dimethylpimelimidate,
dimethylsuberimidate, p-azidephenacylbromide, p-azidephenylglyoxal,
N-hydroxysuccinimydyl-4-azidebenzoate, 4-fluoro-3-nitrophenylazide,
methyl-4-azidebenzimidate, N-5-azide-2-nitrobenzoyloxysuccinimide,
N-succinimydyl-6-(4'-azide-2'-nitrophenylamino)hexanoate, 1,4-benzoquinone,
N-succinimydyl-3-(2'-pyridyldithio)propionate,
sodium N-(4-maleimidebutyryloxy)sulfosuccinimide salt,
sodium N-(6-maleimidecaproyloxy) sulfosuccinimide salt,
sodium N-(8-maleimidecaproyloxy)sulfosuccinimide salt,
sodium N-(11-maleimideundecanoyloxy)sulfosuccinimide salt,
N-[2-(1-piperazinyl) ethyl]maleimide dihydrochloride,
bisdiazobenzidine, hexamethylene diisocyanate,
toluene diisocyanate, hexamethylene diisothiocyanate,
N,N'-ethylene bismaleinimide, N,N'-polymethylene bisiodoacetamide,
sodium 2,4-dinitrobenzenesulfonate salt,
a carbodiimide derivative wherein a diazo-compound or a condensation reagent represented by RN=C=NR (or R'),
N-hydroxysuccinimide, tri-n-butylamine, butylchloroformate, and isobutyl isocyanide.

Examples of a hydrogel include: a polysaccharide selected from the group consisting of agarose, dextran, chitin, chitosan, carrageenan, hyaluronic acid, chondroitin sulfate, alginic acid, starch, and cellulose, or a derivative thereof; and a hydrophilic polymer composed of at least one compound selected from synthetic polycarboxylic acid such as polyacrylic acid and polymethacrylic acid, polyhydroxyalkyl carboxylic ester such as polyHEMA, synthetic polycarboxylic acid amide such as polyacrylamide, an oligomer and a polymer having polyvinl alcohol or ethylene glycol units, polypeptide such as polyglutamic acid, polyaspartic acid, gelatin and collagen, and a dendrimer and/or a derivative of the compound.

A hydrogel is preferably derivatized so as to contain a reactive group such as a hydroxy group, carboxyl, amino, aldehyde, carbonyl, epoxy or vinyl to immobilize a desired physiologically active substance.

A physiologically active substance to be immobilized on the biosensor surface of the present invention is not particularly limited, as long as it interacts with a measurement subject. Examples thereof include an immune protein, an enzyme, a microorganism, a nucleic acid, a low molecular weight organic compound, a non-immune protein, an immunoglobulin binding-protein, a sugar-binding protein, a sugar-recognizing sugar chain, a fatty acid or fatty acid ester, and a polypeptide or oligopeptide capable of binding to a ligand.

Examples of an immune protein include an antibody and a hapten, the antigen of which is a measurement subject. Examples of an antibody to be used include various immunoglobulins such as IgG, IgM, IgA, IgE and IgD. Specifically, when a measurement subject is human serum albumin, an anti-human serum albumin antibody can be used as an antibody. When a pesticide, an insecticide, methicillin resistant *Staphylococcus aureus,* an antibiotic, narcotic, cocaine, heroin, crack or the like is used as an antigen, there can be applied, for example, an anti-atrazine antibody, an anti-kanamycin antibody, an anti-metamphetamine antibody or antibodies against O antigens 26, 86, 55, 111, 157 etc. in enteropathogenic *Escherichia coli*.

The enzyme to be used herein is not particularly limited, as long as it shows activity against a measurement subject or a substance metabolized from the measurement subject. Various enzymes such as oxidoreductase, hydrolase, isomerase, lyase, or synthetase can be used. Specifically, when the measurement subject is glucose, glucose oxidase can be used. When the measurement subject is cholesterol, cholesterol oxidase can be used. Further, when a pesticide, an insecticide, methicillin resistant *Staphylococcus aureus,* an antibiotic, narcotic, cocaine, heroin or crack or the like is used as a measurement subject, enzymes such as acetylcholin esterase, catecholamine esterase, noradrenaline esterase and dopamine esterase, which specifically react with a substance metabolized from such a measurement subject, can be used.

The microorganisms are not particularly limited, and various microorganisms such as *Escherichia coli* can be used.

Any nucleic acid which complementarily hybridizes to a nucleic acid acting as a measurement subject can be used. As a nucleic acid, both DNA (including cDNA) and RNA can be used. Types of DNA are not particularly limited, and any of naturally occurring DNA, recombinant DNA prepared by gene recombination techniques and chemically synthesized DNA can be used.

As a low molecular organic compound, any compound that can be synthesized by a common organic chemical synthetic method can be used. It is preferred to use a compound having a functional group capable of binding to the linker compound of formula I used in the present invention directly or via a crosslinking compound.

The non-immune protein to be used herein is not particularly limited, and avidin (streptavidin), biotin, a receptor etc. can be used.

Examples of the immunoglobulin binding-protein to be used herein include protein A, protein G, and a rheumatoid factor (RF).

Examples of a sugar-binding protein include lectin.

Examples of fatty acid or fatty acid ester include stearic acid, arachidic acid, behenic acid, ethyl stearate, ethyl arachidate, and ethyl behenate.

When the physiologically active substance is a protein such as an antibody or enzyme or a nucleic acid, the substance can be immobilized by using an amino group, a thiol group or the like of the physiologically active substance and allowing such a group to covalently bind to a functional group on a metal surface.

A biosensor surface having a physiologically active substance thus immobilized thereon can be used as a measurement chip for a biosensor for detecting and/or measuring a substance which interacts with the physiologically active substance.

Thus, the present invention provides a method for detecting and/or measuring a substance which interacts with a physiologically active substance immobilized on the biosensor surface, by bringing the measurement chip for a biosensor of the present invention having the physiologically active substance immobilized thereon into contact with a test substance.

For example, a sample containing a substance which interacts with the physiologically active substance can be used as a test substance.

In the present invention, the interaction between a physiologically active substance immobilized on a biosensor surface and a test substance is preferably detected and/or measured by non-electrochemical detection. Examples of non-electrochemical detection include a surface plasmon resonance (SPR) measuring technique, a quartz crystal microbalance (QCM) measuring technique, and a measuring technique employing functionalized surfaces of particles ranging from gold colloid particles to ultrafine particles.

According to a preferred embodiment of the present invention, the measurement chip for a biosensor of the present invention can be used as, for example, a measurement chip for a surface plasmon resonance biosensor which has a metal membrane located on a transparent substrate.

The measurement chip for a surface plasmon resonance biosensor is a chip which is used in a surface plasmon resonance biosensor, and refers to a member comprising a portion for transmitting and reflecting light emitted from the sensor and another portion for immobilizing a physiologically active substance. The member may be fixed to the body of the above sensor, or may be removable.

The phenomenon of surface plasmon resonance is based on that the intensity of monochromatic light reflected from a boundary between an optically transparent substance such as glass and a thin layer of metal is dependent on the refractive index of a sample located at the irradiation side of the metal. Therefore, a sample can be analyzed by measuring the intensity of monochromatic light reflected.

The present invention is described in more detail with reference to the following examples, but the scope of the present invention is not limited to these examples.

### Examples

### (Example 1)

In this example, a chip for immobilizing a physiologically active substance was prepared by using two types of compounds at a mixing ratio as shown in Table 1.

### (1) Preparation of a biosensor chip with inhibited non-specific binding:

50 nm gold vapor-deposited glass plate (BIACORE) where 50 nm gold was vapor deposited on a glass plate (10 mm x 10 mm, thickness 0.2 mm), was prepared as a substrate. The substrate was treated in Model-208 UV-Ozone Cleaning System (TECHNOVISION INC.) for 30 minutes, and the substrate was then immersed in an ethanol solution obtained by mixing 7-carboxyl-1-hexanethiol (Dojin Chemical) and 6-hydroxy-1-hexanethiol (Dojin Chemical) at the mole ratio (total mole concentration, 1 mM) as shown in Table 1, followed by surface treatment at 25°C for 18 hours. Thereafter, the chip was washed at 40°C five times with ethanol, once with a mixed solvent of ethanol and water, and five times with water, to prepare a chip for immobilizing a physiologically active substance. As a comparative example, a chip was prepared using 7-carboxy-1-hexanethiol only.

### (2) Evaluation of performance of a biosensor chip:

This measurement chip was located on a cartridge block of a commercially available surface plasmon resonance biosensor (manufactured by BIACORE, BIAcore 3000).

### (2-a) Measurement of non-specific binding of a protein to a chip for immobilization

Non-specific binding of a protein to a chip for immobilization was measured as follows. The chip for immobilization was reacted with the protein without using an activating agent. It is preferred that the protein is not adsorbed.

10 mM acetate buffer (pH 4.5) of 40 mg/ml protein A was inpoured into the prepared chip for 10 minutes, and the amount of protein A adsorbed was measured. Also, HBS buffer (pH 7) of 15 µg/ml rabbit IgG was inpoured therein for 10 minutes, and the amount of rabbit IgG adsorbed was measured. The flow rate was 10 µL/minute. Resonance signals (RU) after 10 minutes are respectively shown in Table 1 as an index for physical adsorption.

### (2-b) Measurement of protein interaction

Protein interaction was measured as follows.

Protein A was immobilized on a chip for immobilizing a physiologically active substance by pouring 70 µ L of a mixed solution of 1-ethyl-2,3-dimethylaminopropylcarbodiimide (400 mM) and N-hydroxysuccinimide (100 mM) into a measuring cell at a flow rate of 10 µL/minute, and then pouring 10 mM acetate buffer solution (pH 4.5) of 40 mg/ml protein A for 30 minutes. Thereafter, the unreacted component of the immobilized protein A was decomposed with 70 µL of 1M ethanolamine (pH 8), and then 10 µL of 10 mM glycine-hydrochloride buffer (pH 2) was poured in the measuring cell, followed by washing. The optical intensity was measured while pouring rabbit IgG (diluted to 15 µg/ml) at a flow rate of 10 µL/minute for 10 minutes into a measuring cell having protein A immobilized therein, and a resonance signal was determined. Table 2 shows a resonance signal (RU) generated by protein A immobilization and, as an index for the remaining activity of protein A, a ratio of a resonance signal (RU) of bound IgG and a resonance signal (RU) of immobilized protein A.

**Table 1:**

| Non-specific binding of a protein to a chip for immobilization | | | |
|---|---|---|---|
| 7-carboxyl-1-hexanethiol(mM) | 6-hydroxy-1-hexanethiol(mM) | Non-specific binding of Protein A (RU) | Non-specific binding of IgG (RU) |
| 1.0 | 0 | 441 | 70 |
| 0.9 | 0.1 | 305 | 53 |
| 0.5 | 0.5 | 18 | 8 |
| 0.1 | 0.9 | 10 | 4 |

**Table 2:**

| Amount of immobilized Protein A, and ratio of immobilized IgG to Protein A | | | |
|---|---|---|---|
| 7-carboxyl-1-hexanethiol (mM) | 6-hydroxy-1-hexanethiol (mM) | Amount of immobilized Protein A (RU) | IgG(RU)/Protein A(RU) corresponding to the remaining activity of Protein A |
| 1.0 | 0 | 450 | 1.0(normalized) |
| 0.9 | 0.1 | 445 | 1.1 |
| 0.5 | 0.5 | 300 | 1.4 |
| 0.1 | 0.9 | 100 | 1.7 |

From the results in Table 1, it is understood that mixing of 6-hydroxy-1-hexanethiol decreases physical adsorption of a protein to a chip.

From the results in Table 2, it is understood that mixing of 6-hydroxy-1-hexanethiol relatively improves the remaining activity of protein A immobilized on a chip.

### Industrial Applicability

The present invention can provide a method for immobilizing a physiologically active substance on a metal surface in simple processes with high reliability while most of the molecules maintain activity and do not become detached.

## Claims

1. A biosensor surface for immobilizing a physiologically active substance via covalent binding, which comprises a metal surface or metal membrane treated with a mixture containing at least one compound represented by formula (1) and at least one compound represented by formula (2);
X¹-A¹-Y¹ (1)
in formula (1), X¹ represents a functional group capable of covalently binding to a metal surface; A¹ represents a divalent linking group selected from a substituted or unsubstituted amino acid, an aliphatic group, an aromatic group, a heterocyclic group or a combination thereof; and Y¹ represents a functional group capable of binding to a physiologically active substance.
X²-A²-Y² (2)
in formula (2), X² represents a functional group capable of covalently binding to a metal surface; A² represents a divalent linking group selected from a substituted or unsubstituted amino acid, an aliphatic group, an aromatic group, a heterocyclic group or a combination thereof; and Y² represents a functional group capable of improving a performance of a sensor.

2. The biosensor surface according to claim 1 which is used in non-electrochemical detection.

3. The biosensor surface according to claim 1 which is used in a surface plasmon resonance analysis.

4. The biosensor surface according to claim 1 wherein, in formula (1) or (2), X¹ and X² are thiol (-SH) or asymmetric or symmetric disulfide (-SS-).

5. The biosensor surface according to claim 1 wherein, in formula (1), Y¹ is -OH, -COOH, -NH₂, -CHO, -NHNH₂, -NCS, an epoxy group, or a vinyl group.

6. The biosensor surface according to claim 1 wherein, in formula (2), Y² is -OH, -COOH, -NH₂, -SO₃H, a sugar, a nucleic acid, a protein, or a water-soluble polymer.

7. The biosensor surface according to claim 1 wherein the compound represented by formula (1) is HS-(CH₂)ₙ-COOH wherein n represents an integer of 1 to 20 or HS-(CH₂)ₙ-NH₂ wherein n represents an integer of 1 to 20.

8. The biosensor surface according to claim 1 wherein the compound represented by formula (2) is HS-(CH₂)ₙ-OH wherein n represents an integer of 1 to 20, HS-(CH₂)ₙ-NH₂ wherein n represents an integer of 1 to 20, HS-(CH₂)ₙ-SO₃H wherein n represents an integer of 1 to 20, a sugar or sugar derivative having a thiol group, a protein or protein derivative having a thiol group, or a nucleic acid or nucleic acid derivative having a thiol group.

9. A measurement chip for a biosensor which is obtained by covalently binding a physiologically active substance on the biosensor surface of claim 1.

10. A method for detecting and/or measuring a substance which interacts with a physiologically active substance immobilized on the biosensor surface, which comprises a step of bringing the biosensor surface of claim 1 or the measurement chip for a biosensor of claim 9 into contact with a test substance.

11. The method according to claim 10 wherein the interaction between a physiologically active substance immobilized on the biosensor surface and the test substance is detected and/or measured by a non-electrochemical method.

12. The method according to claim 10 wherein the interaction between a physiologically active substance immobilized on the biosensor surface and the test substance is detected and/or measured by surface plasmon resonance analysis.

13. A method for producing a biosensor surface for immobilizing a physiologically active substance via covalent binding which comprises a metal surface or metal membrane treated with a mixture containing at least one compound represented by formula (1) and at least one compound represented by formula (2), which comprises a step of treating a metal surface or metal membrane with a mixture containing at least one compound represented by formula (1) and at least one compound represented by formula (2);
X¹-A¹-Y¹ (1)
in formula (1), X¹ represents a functional group capable of covalently binding to a metal surface; A¹ represents a divalent linking group selected from a substituted or unsubstituted amino acid, an aliphatic group, an aromatic group, a heterocyclic group or a combination thereof; and Y¹ represents a functional group capable of binding to a physiologically active substance.
X²-A²-Y² (2)
in formula (2), X² represents a functional group capable of covalently binding to a metal surface; A² represents a divalent linking group selected from a substituted or unsubstituted amino acid, an aliphatic group, an aromatic group, a heterocyclic group or a combination thereof; and Y² represents a functional group capable of improving a performance of a sensor.

14. A method for immobilizing a physiologically active substance on a metal surface or metal membrane, which comprises steps of: treating a metal surface or metal membrane with a mixture containing at least one compound represented by formula (1) and at least one compound represented by formula (2); and covalently binding a physiologically active substance to the compound represented by formula (1) directly or via a crosslinking compound or hydrogel.
X¹-A¹-Y¹ (1)
in formula (1), X¹ represents a functional group capable of covalently binding to a metal surface; A¹ represents a divalent linking group selected from a substituted or unsubstituted amino acid, an aliphatic group, an aromatic group, a heterocyclic group or a combination thereof; and Y¹ represents a functional group capable of binding to a physiologically active substance.
X²-A²-Y² (2)
in formula (2), X² represents a functional group capable of covalently binding to a metal surface; A² represents a divalent linking group selected from a substituted or unsubstituted amino acid, an aliphatic group, an aromatic group, a heterocyclic group or a combination thereof; and Y² represents a functional group capable of improving a performance of a sensor.

15. The method according to claim 13, wherein, in formula (1) or (2), X¹ and X² are thiol (-SH) or asymmetric or symmetric disulfide (-SS-).

16. The method according to claim 14, wherein, in formula (1) or (2), X¹ and X² are thiol (-SH) or asymmetric or symmetric disulfide (-SS-).

17. The method according to claim 13 wherein, in formula (1), Y¹ is -OH, -COOH, -NH₂, -CHO, -NHNH₂, -NCS, an epoxy group, or a vinyl group.

18. The method according to claim 14 wherein, in formula (1), Y¹ is -OH, -COOH, -NH₂, -CHO, -NHNH₂, -NCS, an epoxy group, or a vinyl group.

19. The method according to claim 13 wherein, in formula (2), Y² is -OH, -COOH, -NH₂, -SO₃H, a sugar, a nucleic acid, a protein, or a water-soluble polymer.

20. The method according to claim 14 wherein, in formula (2), Y² is -OH, -COOH, -NH₂, -SO₃H, a sugar, a nucleic acid, a protein, or a water-soluble polymer.

21. The method according to claim 13 wherein the compound represented by formula (1) is HS-(CH₂)ₙ-COOH wherein n represents an integer of 1 to 20 or HS-(CH₂)ₙ-NH₂ wherein n represents an integer of 1 to 20.

22. The method according to claim 14 wherein the compound represented by formula (1) is HS-(CH₂)ₙ-COOH wherein n represents an integer of 1 to 20 or HS-(CH₂)ₙ-NH₂ wherein n represents an integer of 1 to 20.

23. The method according to claim 13 wherein the compound represented by formula (2) is HS-(CH₂)ₙ-OH wherein n represents an integer of 1 to 20, HS-(CH₂)ₙ-NH₂ wherein n represents an integer of 1 to 20, HS-(CH₂)ₙ-SO₃H wherein n represents an integer of 1 to 20, a sugar or sugar derivative having a thiol group, a protein or protein derivative having a thiol group, or a nucleic acid or nucleic acid derivative having a thiol group.

24. The method according to claim 14 wherein the compound represented by formula (2) is HS-(CH₂)ₙ-OH wherein n represents an integer of 1 to 20, HS-(CH₂)ₙ-NH₂ wherein n represents an integer of 1 to 20, HS-(CH₂)ₙ-SO₃H wherein n represents an integer of 1 to 20, a sugar or sugar derivative having a thiol group, a protein or protein derivative having a thiol group, or a nucleic acid or nucleic acid derivative having a thiol group.
